**Europäisches Patentamt**

**European Patent Office**

**Office européen des brevets**

(19)

(11) Publication number: **0 133 151**
**A2**

# (12) EUROPEAN PATENT APPLICATION

(21) Application number: **84630110.9**

(22) Date of filing: **24.07.84**

(51) Int. Cl.⁴: **A 61 K 7/48**
**A 61 K 33/04, A 61 K 35/78**

(30) Priority: **27.07.83 IL 69353**

(43) Date of publication of application:
**13.02.85 Bulletin 85/7**

(84) Designated Contracting States:
**AT BE CH DE FR GB IT LI LU NL SE**

(71) Applicant: **Berman, Daniel**
**49 Rehov Harav Friedman**
**Tel Aviv(IL)**

(72) Inventor: **Berman, Daniel**
**49 Rehov Harav Friedman**
**Tel Aviv(IL)**

(74) Representative: **Schmitz, Jean-Marie**
**Office Dennemeyer S.à.r.l. 21-25 Allée Scheffer P.O. Box**
**41**
**L-2010 Luxembourg(LU)**

(54) **Skin ointment.**

(57) An ointment for the treatment of skin diseases including an emulsion of extracts of a plurality of herbs, preferably none of the herbs being of the Composite family, selenium rich water, and a carrier. In particular, the carrier includes vaseline, glycerol and paraffin oil.

EP 0 133 151 A2

SKIN OINTMENT

The present invention relates to cream formulations for the treatment of skin diseases in general and, in particular, to cream formulations including herbal extracts having medicinal properties for treatment of skin diseases.

Extracts of various herbs and other plants have long been known to possess healing properties for a variety of ailments. Treatment of these ailments ranges from drinking a broth prepared by boiling portions of the plants in water to  applying whole or crushed leaves directly to the wound.

Mineral waters from various sources around the world,  such as those of the Dead Sea in Israel, are also known to have healing  properties. Treatment of a number of ailments consists of immersion of the body in the mineral waters for varying lengths of time.

There exist a group of recurring skin diseases for which no cure exists and treatment of which has theretofore required application of a preparation of some sort to the  diseased skin at regular intervals for a prolonged period of time. Even then, in many cases success was not assured. The most  common such skin ailment is teenage acne, and others include various eczemas, psoriasis, and so on.

Various chemical compositions have been proposed for the treatment  and protection of the skin. For example, U.S. Patent 4 036 991 to. Stiefel discloses the use of emollient cis-6-Hexadecenoic Acid or derivatives thereof (a fatty acid which constitutes about one third of sebum, the endogenous lubricant produced by the skin ) as a therapeutic or cosmetic supplement for  human skin.

United States Patent 4 229 437 to Likens discloses the use of a plaster containing a composition comprising three parts by volume of bittersweet dried and ground root bark and one part  by volume of zinc chloride (butter of zinc) crystals for the removal of

- 2 -

unwanted lesions or skin growths. The lesion is covered with the plaster and for twenty four hours is kept warm and dry. Pain is experienced for eleven or twelve hours and is accompanied by redness and swelling of the involved area. Once the plaster is removed, the area is bathed twice or three times daily and between washings the lesion is covered with petroleum jelly. This is continued about seven days until the leasion is removed.

United States Patent 4 192 866 to Anderson discloses an anorectal medication for the treatment of hemorrhoids and other anorectal diseases comprising ripe berries of the plant Solanum carolinense dispersed through an application medium. Haimowitz et al. in U.S. Patent 4 169 143 disclose a composition comprising natural or synthetic Vitamin E and a benzoin solution in a pharmacologically acceptable carrier for treatment of hemorrhoid symptoms. And Homan, in U.S. Patent 3 935 310, provides a composition for treating hemorrhoids comprising a mixture of the powdered or chipped limbs or roots of the shrub Celastrus scandens heated with an animal fat or a vegetable oil. Preferably a topical anesthetic is also added to the composition.

U.S. Patent 4 235 889 to Evers discloses a pharmaceutical composition for the treatment of psoriasis tinea and eczemas, comprising coconut oil, palm kernel oil, an extract of Laurus nobilis (Linn.) and an emulsifier. Tubery describes an alcoholic extraction product of the root of the shrubby species known as Securidaca longipedonculata for the treatment of skin diseases, particularly eczema and sporiasis, in U.S. Patent 3 584 123. Comfort tells of treatment of psoriasis by applying a natural fluid extract of walnut hulls to the afflicted areas of the skin , in U.S. Patent 3 562 389.

Other medical compositions employing natural substances include those described in U.S. Patents 4 353 896 (oil of eucalyptus) against sprains and athletic injuries, 4 154 823 (PABA and other members of the vitamin B complex) for sunburn and other burns, 4 021 578 (grapefruit pulp) as a cosmetic and antibacterial agent, and U.S. Patent 3 773 931 (a pharmacologically effective substance isolated from Cabucala madagascariensis having a high hypotensive effect.

It will be appreciated that these formulations employ extracts from one or at most two different plants along with a suitable carrier or emulsifier. The majority include non-natural components, i.e., synthetics or chemicals which are foreign to the human body as active ingredients or emulsifiers.

It is therefore an object of the present invention to provide a medicament for the treatment of a variety of skin ailments composed of only natural ingredients, in particular, herbal extracts and selenium rich water in a mixture of carriers, which does not suffer from the disadvantages of prior art medications.

There is thus provided in accordance with the present invention an ointment for the treatment of skin diseases including an emulsion of extracts of a plurality of herbs, preferably none of the herbs being of the Composite family, selenium rich water, and a carrier. In particular, the carrier includes vaseline , glycerol and paraffin oil.

According to a preferred embodiment, the emulsion includes 0.4 - 0.6% by volume of extracts and 15-20% by volume of selenium rich water, and the carrier includes medical grade vaseline, glycerol and paraffin oil in a 3:1:1 ratio. Further according to a preferred embodiment; the selenium rich water includes 10-32 ppm selenium, and most preferably 23-27 ppm selenium.

In accordance with a preferred embodiment of the invention, the extracts are extracts of herbs selected from the group consisting of Rosmarinus officinalis, Hypericum perforatum, Hedera helix, Capsicum minimum, Cucumis savitus, Fucus crispus, Hamamelis virginia, Ulmaria, Aesculus hippocastanum and Salvia officinalis.

There is further provided in accordance with an embodiment of the present invention an ointment for treatment of hemorrhoids and varicose veins comprising an emulsion including extracts of each of Rosmarinus officinalis, Hypericum perforatum, Hedera helix, Capsicum minimum, Cucumis savitus, Fucus crispus, Hamamelis virginia, Aesculus hippocastanum and Salvia officinalis, selenium rich water and a carrier, the carrier most preferably including medical grade paraffin oil, glycerol and vaseline.

There is still further provided in accordance with an embodiment of the present invention an ointment for treatment of psoriasis comprising an emulsion including extracts of each of Rosmarinus officinalis, Hypericum perforatum, Hedera helix, Cucumis savitus, Fucus crispus, Hamamelis virginia, Ulmaria, selenium rich water and a carrier, the carrier most preferably including medical grade paraffin oil, glycerol and vaseline.

There is additionally provided in accordance with an embodiment of the present invention an ointment for treatment of skin diseases comprising an emulsion including extracts of each of Rosmarinus officinalis, Hypericum perforatum, Hedera helix, Cucumis savitus, Fucus crispus, Hamamelis virginia, Ulmaria, Salvia officinalis, selenium rich water and a carrier, the carrier most preferably including medical grade paraffin oil, glycerol and vaseline.

According to a preferred embodiment of the present invention, the ointment includes equal parts of each of the herbal extracts.

There is also provided a method for treating skin diseases including topically applying any of the above ointments to the diseased area a plurality of times

- 5 -

per day until the symptoms of the disease disappear.

The compositions of the present invention are emulsions of herbal extracts from a number of herbs, natural selenium rich water and a carrier. It is a particular feature of the present invention that the use of a plurality of herbs in the composition results in the amines in the extracts acting as emulsifiers so that no additional, synthetic emulsifier is required. Furthermore, instead of a final concentration of 2-20% of extract which is required in existing compositions to achieve clinical results, only 0.4 - 0.6% is required in the compositions of the present invention.

The herbs which have been found to be particularly effective in the treatment of a wide range of skin diseases are Rosmarinus officinalis and Salvia officinalis, of the Labiatae family, Hypericum perforatum, of the Hypericaceae family, Hedera helix, of the Araliaceae family. Capsicum minimum, family Solanaceae, Cucumis savitus, family Cucumbitaceae, Fucus crispus, Gigartinaceae family, Hamamelis virginia, Hamamalidaceae family, Ulmaria or Meadow Sweet, of the Rosaceae family, and Aesculus hippocastanum, of the Hippocastanaceae family. It is anticipated that other plants in these families having known medicinal properties will also be useful in the compositions of the present invention.

It has been found that plants of the Composite family, such as the dandelion (Taraxacum officinale), are operative in the compositions of the present invention, but occasionally result in an allergic reaction by the person being treated. They are therefore not preferred components.

Plant extracts which may be employed in the compositions of the present invention are conventional extracts, generally 12-15% in alcohol. The plants may be extracted according to any known extraction technique. Suitable extracts are commercially available from a number

- 6 -

of companies, for example, Exsymol, a corporation of Monaco.

The second main component is selenium rich water. According to a preferred embodiment of the invention, the water is natural spring water such as from natural springs around the Dead Sea, Israel. Unlike prior art compositions, distilled water is not required or desirable . In fact, the hardness of the water employed in the present invention may be about 14 or $15^{\circ}$ on the French scale. The selenium content of normal tap water is on the order of 5 to 7 ppm. The selenium content required in the compositions of the present invention to achieve clinical results is 10 ppm to 32 ppm. Preferably, the water contains 23 to 27 ppm selenium. Generally, with more than 32 ppm selenium, the water becomes too hard to emulsify.

The carrier of the present invention preferably consists of a mixture of medical grade vaseline, medical grade glycerol and medical grade paraffin oil. Any commercially available vaseline, glycerol or paraffin oil of medical grade will be operative in the present invention.

It is a particular feature of the present invention that the carrier consists of a mixture of materials of varying viscosities, rather than vaseline or oil alone, as in prior art compositions. This permits preparation of a fine homogeneous emulsion of a consistency convenient for application. According to a preferred embodiment of the invention, the carrier consists of three parts vaseline by volume, one part glycerol and one part paraffin oil. The final concentration of carrier in the composition will be on the order of 75-80%.

In order to more clearly illustrate the compositions of the present invention, the following Examples are presented. It will be appreciated, however, that these are merely presented by way of illustration and that any number of equally useful and effective compositions may be made by varying the specific herbal extracts included in the

- 7 -

composition or changing the relative quantities thereof.

Example 1

3.0 - 3.5 ml each of herbal extracts of Rosmarinus officinalis, Hypericum perforatum, Hedera helix, Capsicum minimum, Cucumis savitus, Fucus crispus, Hamamelis virginia, Aesculus hippocastanum and Salvia officinalis manufactured by Exsymol, Monaco, were mixed together . One half of this mixture (15ml) was emulsified with 480 ml medical grade vaseline, 160 ml medical grade glycerol and 160 ml medical grade paraffin oil ( a ratio of 3:1:1 by volume) to produce 815 ml of an emulsion having a concentration of 0.2% herbal extracts.

The remaining herbal extracts, 15 ml total, were emulsified with 223 ml natural selenium rich water from the natural springs of Ein Gedi, Israel to a concentration of 0.8% extracts.

815 ml of the first emulsion were mixed with 238 ml of the second emulsion and homogenized to a fine, homogeneous emulsion. The yield was 1 kg of ointment at a final concentration of 0.4% herbal extracts.

Example II

The procedure of Example I was followed with equal parts of herbal extracts of Rosmarinus officinalis, Hypericum perforatum, Hedera helix, Cucumis savitus, Fucus crispus, Hamamelis virginia and Ulmaria of Exsymol, Monaco. One half the mixture was emulsified with the carrier while the other half was emulsified with the water. The two emulsions were homogenized together to yield 1 kg of ointment at a concentration of 0.4% herbal extracts.

Example III

The procedure of Example I was followed utilizing equal parts 4.5 -5.3 ml each , of Rosmarinus officinalis, Hypericum perforatum, Hedera helix, Cucumis savitus, Fucus crispus, Hamamelis virginia, Ulmaria and Salvia officinalis of Exsymol, Monaco. One half the mixture, 20ml, was emulsified with 795 ml of the carrier (a 3:1:1 ratio

- 8 -

by volume) while the other half, 20 ml, was emulsified with 218 ml water. The two emulsions were homogenized together  to yield 1 kg of ointment at a concentration of 0.6% herbal  extracts.

Example IV

The ointments produced by the methods of Examples I,II and III were tested for clinical efficacy in relieving symptoms of anal piles (hemorrhoids) under the guidance of a lincensed  physician. One hundred patients suffering from anal piles complaints were treated by applying one of the ointments topically to the injured tissue three times daily for a period of up to seven days. 50 patients  were treated with the ointment of Example I, 25 patients with the ointment of Example II, and 25 with the ointment of Example III.

Five to seven days after commencement of the treatment, the patients were examined. Clinical results were considered positive if all the following conditions were met at that examination:

1. Disappearance of inflammatory state;

2. Shrinkage of the swollen tissue; and

3. Disappearance of subjective complaints of irritation and discomfort.

The test results are set out in the following Table A.

Table A

| Preparation | Number of Patients | % Positive Clinical Results |
| --- | --- | --- |
| I | 50 | 90 (45 patients) |
| II | 25 | 72 (18 patients) |
| III | 25 | 68 (17 patients) |

Example V

The ointments produced by the methods of Examples I, II and III were tested for clinical efficacy in relieving symptoms of varicose veins under the guidance of a licensed physician. One hundred patients suffering from varicose veins were treated by applying one of the  ointments topically to the skin about the varicose veins three times daily for a period of up to ten days.

50 patients were treated with the ointment of Example I, 25 patients with the ointment of Example II, and 25 with the ointment of Example III.

Seven to ten days after commencement of the treatment, the patients were examined. Clinical results were considered positive if all the following conditions were met at that examination:

1. Disappearance of inflammatory state;

2. Shrinkage of swollen tissue;

3. Healing of wounds; and

4. Disappearance of subjective complaints of itching and locan pains.

The test results are set out in the following Table B.

Table B

| Preparation | Number of Patients | % Positive Clinical Results |
|---|---|---|
| I | 50 | 82 (41 patients ) |
| II | 25 | 68 (17 patients) |
| III | 25 | 64 (16 patients) |

Example VI

The ointments produced by the methods of Examples I, II and III were tested for clinical efficacy in relieving symptoms of various kinds of skin eczemas and infections including seborrheic dermatitis , bacterial and fungal infections, burns and acne vulgaris under the guidance of a licensed physician. Three hundred patients suffering from various skin ailments were treated by applying one of the ointments topically to the diseased or injured tissue three times daily for a period of up to fifteen days. 100 patients were treated with the ointment of Example I, 100 patients with the ointment of Example II, and 100 with the ointment of Example III.

Seven to fifteen days after commencement of the treatment, the patients were examined, clinical results were considered positive if all the following conditions were met at that examination:

1. Disappearance of inflammatory state;
2. Disappearance of plaques;
3. Healing of wounds; and
4. Disappearance of subjective complaints of itching and local pains.

The test results are set out in the following Table C.

Table C

| Preparation | Number of Patients | % Positive Clincial Results |
|---|---|---|
| I | 100 | 61 (61 patients) |
| II | 100 | 85 (85 patients) |
| III | 100 | 63 (63 patients) |

Example VII

The ointments produced by the methods of Examples I, II and III were tested for clinical efficacy in relieving symptoms of psoriasis under the guidance of a licensed physician. One hundred patients suffering from psoriasis were treated by applying one of the ointments topically to the diseased tissue three times daily for a period of up to twenty days. 25 patients were treated with the ointment of Example I, 25 patients with the ointment of Example II, and 50 with the ointment of Example III.

Five to twenty days after commencement of the treatment, the patients were examined. Clinical results were considered positive if all the following conditions were met at that examination :

1. Disappearance of inflammatory state;
2. Disappearance of plaques;
3. Disappearance of subjective complaints of pruritic pains; and
4. Healing of wounds.

The test results are set out in the following Table D.

- 11 -

Table D

| Preparation | Number of Patients | % Positive Clinical Results |
|---|---|---|
| I | 25 | 64 (16 patients ) |
| II | 25 | 72 (18 patients) |
| III | 50 | 80 (40 patients) |

Treatment of these various skin ailments by topical application of the ointments of the present invention is simple, it produces relatively fast results, and it acts rapidly to relieve pain and itching.

It will be appreciated by those skilled in the art that the invention is not limited to what has been described and exemplified hereinabove. Rather, the scope of the invention is limited solely by the following claims.

CLAIMS:

1.      An ointment for the treatment of skin diseases comprising an emulsion comprising:

extracts of a plurality of herbs;

selenium rich water; and

a carrier.

2.      An ointment according to claim 1 and wherein said carrier comprises :

vaselines;

glycerol; and

paraffin oil.

3.      An ointment according to claim 1 and wherein said emulsion comprises 0.4 -0.6% by volume of herbal extracts and 15-20% by volume of selenium rich water.

4.      An ointment according to claim 1, and wherein said carrier comprises medical grade vaseline, medical grade glycerol and medical grade paraffin oil in a 3:1:1 ratio by volume.

5.      An ointment according to claim 1 and wherein said selenium rich water comprises 10-32 ppm selenium.

6.      An ointment according to claim 1 and wherein said selenium rich water comprises 23 to 27 ppm selenium.

7.      An ointment according to claim 1 and wherein said extracts are extracts of herbs selected from the group consisting of Rosmarinus officinalis, Hypericum perforatum, Hedera helix,  Capsicum minimum, Cucumis savitus, Fucus crispus, Hamamelis virginia, Ulmaria, Aesculus hippocastanum and Salvia officinalis.

8.      An ointment for treatment of hemorhhoids and varicose veins comprising an emulsion comprising:

extracts of each of Rosmarinus officinalis, Hypericum perforatum, Hedera helix, Capsicum minimum, Cucumis savitus, Fucus crispus, Hamamelis virginia, Aesculus hippocastanum and Salvia officinalis;

selenium rich water; and

a carrier.

9.          An ointment for treatment of psoriasis comprising an emulsion comprising:
          extracts of each of Rosmarinus officinalis,
Hypericum perforatum, Hedera helix, Cucumis savitus,
Fucus crispus, Hamamelis virginia and Ulmaria;
          selenium rich water; and
          a carrier.

10.          An ointment for treatment of eczemas and skin
infections comprising an emulsion comprising:
          extracts of each of Rosmarinus officinalis,
Hypericum perforatum,  Hedera helix, Cucumis savitus,
Fucus crispus, Hamamelis virginia, Ulmaria and Salvia
officinalis;
          selenium rich water; and
          a carrier.

11.          An ointment according to any of claims 8 to 10
and wherein said carrier comprises medical grade
paraffin oil,  medical grade glycerol and medical grade
vaseline.

12.          An ointment according to any of claims 8 to 11
and wherein said selenium rich water comprises 10 to 32
ppm selenium.

13.          An ointment according to any of claims 8 to 11
and wherein said selenium rich water comprises 23 to 27
ppm selenium.

14.          An ointment according to any of claims 8 to 13
and wherein said carrier comprises medical grade paraffin
oil, medical grade glycerol and medical grade vaseline
in a 3:1:1 ratio by volume.

15.          An ointment according to any of the preceding
claims and wherein said ointment comprises equal parts
of each of said herbal extracts.

16.          A method of treating skin ailments comprising
the steps of topically applying the ointment of any of
the preceding claims  to the diseased area a plurality
of times per day until the symptoms of the ailment disappear.

17.       A method of treating hemorrhoids comprising the steps of topically applying the ointment of claim 8 to the injured tissue a plurality of times per day until the hemorrhoids disappear.

18.       A method of treating varicose veins comprising the steps of topically applying the ointment of claim 8 to the skin about the varicose veins a plurality of times per day until the varicose veins disappear.

19.       A method of treating eczemas and skin infections comprising the steps of topically applying the ointment of claim 9 to the diseased area a plurality of times per day until the  symptoms of the ailment disappear.

20.       A method of treating psoriasis comprising the steps of  topically applying the ointment of claim 10 to the diseased area a plurality of times per day until the symptoms of the ailment disappear.

21.       An ointment according to any of claims 1-15 and wherein a synthetic emulsifier is not present.

22.       An ointment substantially as described or exemplified hereinabove.

23.       A method  substantially as described or exemplified hereinabove.